# EUROPEAN PATENT SPECIFICATION

(11) **EP 2 223 699 B1**
(45) Date of publication and mention of the grant of the patent: **20.08.2014**
(21) Application number: 08857855.4
(22) Date of filing: 08.12.2008
(51) Int. Cl.: A61K 38/51, A61P 17/02

(54) **RADICAL THERAPEUTIC AGENT FOR KELOID AND HYPERTROPHIC SCAR**
RADIKALES THERAPEUTISCHES MITTEL GEGEN KELOID- UND HYPERTROPHE NARBEN
AGENT THÉRAPEUTIQUE RADICAL POUR CICATRICE CHÉLOÏDE ET HYPERTROPHIQUE

(30) Priority: 07.12.2007 JP 2007317294
(43) Date of publication of application: 01.09.2010
(73) Proprietor: SEIKAGAKU CORPORATION, Chiyoda-ku Tokyo 100-0005 (JP); Suzuki, Shigehiko, Kyoto 606-8507 (JP)
(72) Inventor: SUZUKI, Shigehiko, Kyoto-shi Kyoto 606-8507 (JP); NAITO, Motoko, Kyoto-shi Kyoto 606-8507 (JP); IKEDA, Mika, Kyoto-shi Kyoto 606-8507 (JP)
(74) Representative: Elsy, David
(86) International application number: PCT/JP2008/072279
(87) International publication number: WO 2009/072654

(56) References cited:
- WO-A2-01/39795
- JP-T- 2004 504 262
- YAMAGATA T ET AL: "Purification and properties of bacterial chondroitinases and chondrosulfatases.", THE JOURNAL OF BIOLOGICAL CHEMISTRY 10 APR 1968 LNKD- PUBMED:5647268, vol. 243, no. 7, 10 April 1968 (1968-04-10), pages 1523-1535, XP002612430, ISSN: 0021-9258
- SEIKAGAKU JITEN: 'Kabushiki Kaisha Tokyo Kagaku Dojin', 20 November 1998 page 556, XP008128534
- MIKA IKEDA ET AL.: 'Keloid ni Okeru Dansei Sen'i no Ijo to Glycosaminoglycan no Kajo Chikuseki' DAI 14 KAI JAPAN SOCIETY OF PLASTIC AND RECONSTRUCTIVE SURGERY KISO GAKUJUTSU SHUKAI PROGRAM SHOROKUSHU 2005, page 93, XP008128533

## Description

### TECHNICAL FIELD

The present invention relates to an elastic fiber formation promoting agent.

### BACKGROUND ART

The abbreviations used herein are as follows.
GAG: glycosaminoglycan
CS: chondroitin sulfate
CS-A: chondroitin sulfate A
CS-B: chondroitin sulfate B
CS-C: chondroitin sulfate C
CSPG: chondroitin sulfate proteoglycan
GAGase: glycosaminoglycan lyase
CSase: chondroitinase (chondroitin sulfate lyase)
CSase-ABC: chondroitinase ABC
CSase-B: chondroitinase B
CSase-AC: chondroitinase AC

Hypertrophic scars and keloids are characterized by, so to speak, an abnormality in wound healing, in which a fibrous tissue called "scar tissue" is formed, in the process of wound healing occurred on the skin, without regeneration of original normal tissue (non-Patent Documents 1 and 2). While a hypertrophic scar occur as a result of interference in wound healing, such as a large and deep wound, infection, contact with a foreign body or inappropriate suture, a keloid may arise from a very minor wound such as an insect bite or a puncture by a vaccine shot. Keloids are characterized by their growth beyond the boundaries of the initial injury wound site (non-Patent Documents 2 and 3). However, hypertrophic scars and keloids are common in that both of their lesion portions are a red-colored elevated lesion, which is primarily characterized by an excessive accumulation of extracellular matrix and cell proliferation. The lesion portions are extremely hard, thereby markedly restricting the elasticity of the skin. Because of this, these affected areas not only accompany pain, but also cause a functional impediment if located over a joint, such as restriction of the range of the joint motion. The lesion portions may also cause growth disorder if the patient is a child; therefore, the treatment of hypertrophic scars and keloids is important not only from simple cosmetic reasons, but also from the functional perspectives. However, there is no appropriate model for animal experimentation for hypertrophic scars and keloids, thus the clarification of the etiology and pathology has not seen much progress up to present.

The treating methods that are currently employed are as follows.

### 1) Pressure-fixation therapy

In general, a sponge with an adhesive agent on one side (such as Reston or Fixton (registered trademarks)) is directly patched on the surface of the lesion portion, and this is pressed with surgical tape and fixed (non-Patent Documents 4 to 6). In cases where the lesion portion is located on a movable part, on top of the surgical tape, the lesion portion is further wrapped with a dressing or supporter strap, or a girdle or corset is wore (non-Patent Document 7). Such pressuring flattens the lesion portion and relieves pain and itchiness; however, the termination of such pressuring results in re-swelling of the lesion portion and reemergence of pain and itchiness.

### 2) Surgical therapy:

When the lesion portion is small or in the case of a narrow hypertrophic scar, the symptoms are relieved by excision or appropriate plastic surgical suture thereof. However, pressure-fixation therapy is required for about three months even after the suture removal (non-Patent Document 8). Further, the surgical therapy is not applicable to a wide lesion portion or hypertrophic scar extending over a large area, such as a thermal injury. Furthermore, in the case of a keloid, even if the lesion portion was once flattened, the lesion portion would reoccur therefrom, and would regenerate a lesion portion extending over an area larger than the one prior to the surgery (non-Patent Document 16). In order to prevent this recurrence, radiotherapy is necessary after the lesion removal; however, radiotherapy does nothing more than lowering the recurrence rate; therefore, this therapy is not a radical treatment (non-Patent Document 16).

### 3) Therapy using a cover material:

Since the report by Perkins et al. (non-Patent Document 9), hypertrophic scars and keloids have been treated by patching a silicone gel sheet, and in some cases, the symptoms are improved. The mechanism of action thereof is believed to be the moisturizing action; however, the details thereof are not clear (non-Patent Document 10). A hydrocolloid covering material has also been used in place of silicone gel. Yet, this therapy does nothing more than improving the symptoms and the effects thereof are not stable. In addition, there have been many cases where even an improvement of the symptom was not observed; therefore, this therapy is not a radical treatment.

### 4) Pharmacotherapy:

Steroid drugs (triamcinolone solution) have been topically injected into the lesion portion of a hypertrophic scar and keloid (non-Patent Document 11). This injection not only flattens the lesion portion, but also fades the redness and alleviates the itchiness. However, depending on the dosage, systemic side effects, such as atrophoderma, hypopigmentation, excessive pigmentation and telangiectasia, may occur; therefore, these steroid drugs cannot be administered over a prolonged period. Further, the injection is not applicable to lesions extending over a large area as well (non-Patent Document 12 and non-Patent Document 16). Furthermore, women may also experience a side effect such as menstrual irregularity, even at a small dosage. In addition, since recurrence is observed in many patients upon the termination of steroid drug injection, steroid drugs are not a radical treatment, and patients are forced to attend a hospital for a prolonged period. The action of a steroid drug is based on an anti-inflammatory effect, such as cytokine inhibition, and a subsequent reduction in the number of keloid cells (large fibroblasts having traits characteristic to keloids). The recurrence in the steroid therapy indicates that a mere induction of a reduction in the number of keloid cells by anti-inflammatory effect does not lead to a radical care (non-Patent Document 16). There is also a method in which a tape agent containing a steroid such as betamethazone or fludroxycortide is patched other than the topical injection; however, the effects thereof do not go beyond the improvement of the symptoms and are unstable. Further, some patients may develop skin rushes by such tape. As a medication therapy, tranilast is used (non-Patent Document 13). This also does nothing more than improving the symptoms such as itchiness in some cases, and in many cases, it has to be taken for not less than 3 months.

In this manner, these conventional therapies for hypertrophic scars and keloids, although they are called "treatment", are nothing more than supportive measures. That is, the conventional therapies do nothing more than simply flattening the torous lesion portion in a hypertrophic scar and keloid and temporarily improving the symptoms. In the medical field concerning hypertrophic scars and keloids, there has not been a concept per se to radically treat a lesion tissue by inverting it to a normal tissue. Consequently, up to the present date, the world has never seen a therapeutic agent which normalizes hypertrophic scars and keloids (i.e. which allows hypertrophic scars and keloids to recover to the normal tissue condition), that is, a radical therapeutic agent.

As the major constituent of human skin, collagens, elastic fibers and GAGs are known. In hypertrophic scars and keloids, excessive accumulation of Type I, Type III and Type VI collagens (non-Patent Documents 14 and 15) and deficiencies in the normal structure of elastic fibers (non-Patent Documents 17 and 18) have been reported. Elastic fibers, whose major component is elastin, are fibers that, like a rubber and spring, are highly elastic and readily stretch, while they are restored back to their original state when the force is removed. The details of the mechanism of elastic fiber formation are not clear; however, it is believed that elastic fibers are formed when elastin proteins are deposited and cross-linked around a fiber called microfibril. Fibrillin-1 is known as one of the major components of microfibril. In the extracellular matrix of the lesion tissue of hypertrophic scars and keloids, there is a deficiency in the deposition and cross-linking of elastins to fibrillin-1 (non-Patent Document 17).

Patent Document 1 describes that CSases are used to reduce the size of the fibrous tissue, based on the results showing that the CSase-B and CSase-AC inhibited the fibroblast proliferation. However, even if this is to be applied to keloids, it would not provide something beyond the conventional concept of flattening the lesion portion by inhibiting the proliferation of fibroblasts. In view that steroid drugs, which have an inhibitory effect on cell proliferation, cannot be used for a radical treatment of hypertrophic scars and keloids and that keloids and the like reoccur upon the termination of steroid drug administration, normalization of the lesion portion cannot be expected by simply inhibiting the proliferation of fibroblasts.

The tissue of the lesion portion of a keloid patient is different from the lesion tissue of a psoriasis patient or the dermal tissue of a psoriasis model animal (mouse), and is characterized by intricate dense collagen fiber bundles composed of abnormally deposited collagens, hyalinization of these collagen fiber bundles and abnormal accumulation of CSPGs to the extracellular matrix. Further, as a characteristic of keloid tissues, keloid tissues are known to be deficient in elastic fibers (fibers formed by deposition and cross-linking of elastin to fibrillin-1), which are found in normal tissues of human.

In animals other than human, wounds are healed without developing a keloid or the like; therefore, as described in the above, even a test system capable of evaluating the effect to radically treat keloids has not been developed up to the present date. Consequently, there has not been even a concept of radical treatment, in which, as in the present invention, the lesion tissue itself of a keloid and the like is recovered to a normal tissue. That is, it has been considered that such a radical treatment is impossible.
Patent Document 1: JP-A-2004-504262
Non-Patent Document 1: Abergel, R. P., Pizzurro, D. Meeker, C. A., et al. (1985) Biochemical composition of the connective tissue in keloids and analysis of collagen metabolism in keloid fibroblast cultures. J. Invest. Dermatol. 84, 384-390
Non-Patent Document 2: Heenan, P. J. (1997) Tumors of the Fibrous Tissue Involving the Skin. In: Lever's Histopathology of the Skin (eds Elder, D., et al), pp. 847-887. East Washington Square, Philadelphia, Pennsylvania: Lippincott-Raven Publishers.
Non-Patent Document 3: Ooura, T. et al. (1993) Definition and Classification of Keloid Hypertrophic Scar. Japanese Journal of Plastic Surgery 36: 265-274
Non-Patent Document 4: R. Fujimori: Pressure therapy of keloids. J. of Operation, 44:3-13, 1990
Non-Patent Document 5: Costa AM, et al.: Mechanical Force Induce Scar Remodeling: Am J Pathol. 155: 1671-1679, 1999
Non-Patent Document 6: Suzuki, S. and Naito, M.: Treatment of keloids and hypertrophic scars. Japan Medical Journal, 4516: 29-32, 2003
Non-Patent Document 7 : Suzuki, S.: Treatment of cicatricial keloid. MB Derma, 67: 134-138,2002
Non-Patent Document 8: Suzuki, S.: Operation: Operation of keloid and/or hypertrophic scar. 50: 1557-1561, 1996
Non-Patent Document 9: Perkins K, et al.: Silicone gel: a new treatment for burn scars and contractures. Burns 9; 201-204, 1983
Non-Patent Document 10: Ooura, T. et al.: Results of Experimental Use of Silicone Gel Sheet for Treatment of Hypertrophic Scar and Keloid. Journal of Clinical Therapeutics and Medicines 14; 2921-2937,1998
Non-Patent Document 11: Maguire H C : Treatment of keloids with triamcinolone acetonide injected intralesionnally, JAMA, 192: 325-329, 1956
Non-Patent Document 12: Suzuki, S. and Fujimori R. Treatment of itching of hypertrophic scar and keloid. MB Derma, 30:14-19, 1999
Non-Patent Document 13: Namba, Y. et al.: Clinical evaluation of tranilast on keloid and hypertrophic scar. Japanese Journal of Burn Injuries 18:30-45,1992
Non-Patent Document 14 : Peltonen, J., Hsiao, L. L., Jaakkola, S. et al. (1991) Activation of collagen gene expression in keloids: co-localization of type I and VI collagen and transforming growth factor-beta 1 mRNA. J. Invest. Dermatol. 97: 240-248
Non-Patent Document 15: Naitoh, M., Hosokawa, N., Kubota, H. et al. (2001) Upregulation of HSP47 and collagen type III in the dermal fibrotic disease, keloid. Biochem. Biophys. Res. Commun. 280: 1316-1322
Non-Patent Document 16: Robles DT, Moore E, Draznin M, Berg D. Keloids: Pathology and management. Dermatology Online Journal 13(3): 9 (2007)
Non-Patent Document 17: N.V. Kamath, A. Ormsby, W.F. Bergfeld, and N.S. House, A light microscopic and immunohistochemical evaluation of scars. J Cutan Pathol 29 (2002): 27-32
Non-Patent Document 18: K.S. Bhangoo, J.K. Quinlivan, and J.R. Connelly, Elastin fibers in scar tissue. Plast. Reconstr. Surg. 57 (1976): 308-13.

### SUMMARY OF INVENTION

The object of the present invention is to provide a therapeutic agent which radically cures (normalizes) a hypertrophic scar and keloid, which are refractory disorders unique to human, by allowing them to recover to the normal tissue condition.

The present inventors intensively studied to solve the above-described problems and, before the rest of the world, succeeded in developing a test system capable of correctly evaluating the therapeutic effects on keloid patients. This system enabled to evaluate even the process of the tissue normalization which leads to a radical treatment of keloids and the like, not to mention a reduction in the tissue volume of keloids and the like. Here, the process of normalization refers to regeneration of the elastic fiber formation in a keloid tissue, a reduction in the number of abnormally proliferated keloid cells, a reduction in the excessively accumulated collagen fiber bundles, disappearance of hyalinization, a reduction in the keloid tissue and the like. This novel test system includes a method in which the lesion tissues of a keloid patient having a size of 5 mm × 5 mm are collected, and using a precise technique, for example, a combination of production of a minimum-sized subcutaneous pockets and anchoring sutures, the collected lesion tissues are implanted and fixed subcutaneously on the back of a nude mouse. This method enabled the lesion tissues of the keloid patient to maintain the characteristics of keloid for a prolonged period within the corium of an animal skin, thereby allowing the evaluation of the therapeutic effects by administration of a test substance to the lesion portion.

In addition, in relation to the normalization of the tissue of a keloid or the like, the present inventors developed an assay for elastic fiber formation (elastogenesis assay), which is an *in vitro* evaluation system. This test system is one which is capable of measuring the inhibitory property of elastic fibers (fibers formed by deposition and cross-linking of elastin to fibrillin-1) by a test substance. Moreover, using these test systems, the present inventors further intensively studied and as a result, discovered that an enzyme which degrades CS-A, CS-B and CS-C offers a novel approach for promoting the formation of elastic fibers and for radical treatment of keloids and hypertrophic scars, thereby completing the present invention.

That is, the summary of the present invention is as follows.
*(1) In vitro* use of chondroitinase ABC for promoting elastic fiber formation.
(2) The *in vitro* use according to (1), characterized in that said chondroitinase ABC is derived from *Proteus vulgaris.*
(3) Chondroitinase ABC for use in the radical treatment of keloids and/or hypertrophic scars.
(4) The chondroitinase ABC according to (3), characterized in that an association between elastin and fibrillin is promoted by said enzyme.
(5) The chondroitinase ABC according to (3), characterized in that the chondroitinase ABC is derived from *Proteus vulgaris.*

There is also described a use of the enzyme which degrades CS-A, CS-B and CS-C in the production of the elastic fiber formation promoting agent, as well as a use of the enzyme which degrades CS-A, CS-B and CS-C in the production of the radical therapeutic agent for keloids and/or hypertrophic scars.

### BRIEF DESCRIPTION OF DRAWINGS

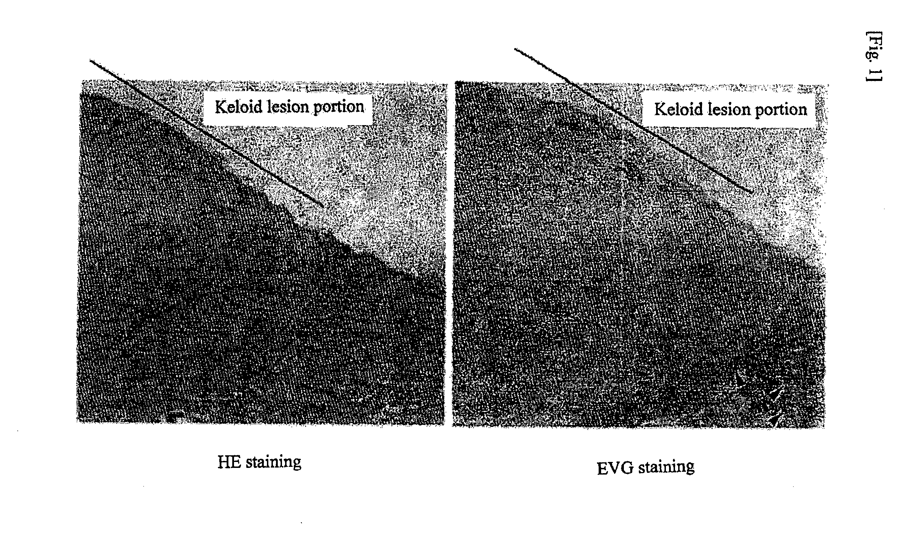

[Fig. 1] Fig. 1 are figures (photographs) showing the result of observation under a microscope on a skin tissue of a keloid patient containing a lesion portion and normal skin part. The region indicated with an oblique line is the keloid lesion portion, and the structurally different adjacent part is the normal skin part. The figure on the left shows a specimen subjected to hematoxylin and eosin (HE) staining, while the figure on the right shows a specimen subjected to Elastica-van Gieson (EVG) staining. The EVG staining is a method which can stain elastic fibers in black.

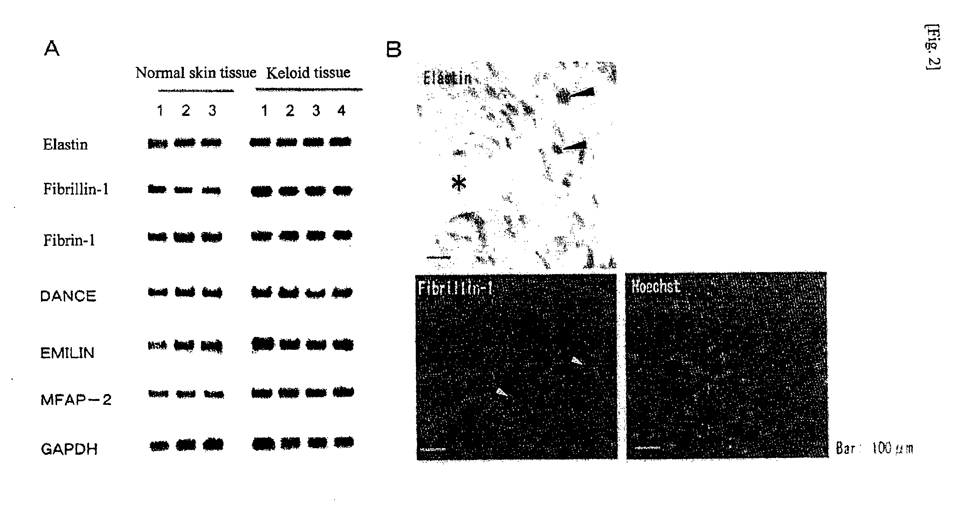

[Fig. 2] Fig. 2 are figures (photographs) showing the results of the expression of elastic fiber constituents in a keloid tissue and normal skin tissue. Fig. 2A is a figure (photograph) showing the results of electrophoresis of RT-PCR-amplified mRNAs of elastic fiber constituents in both of the tissues. Fig. 2B are figures (photographs) showing the expressions of the proteins of elastic fiber constituents in keloid tissue and the existence of localization thereof in the extracellular matrix, which were detected by immunohistochemical staining. The upper figure of Fig. 2B is a specimen subjected to elastin staining. The lower left figure of Fig. 2B is a specimen subjected to fibrillin-1 staining. The lower right figure of Fig. 2B is a specimen of which each of the keloid cell nuclei was stained with Hoechst.

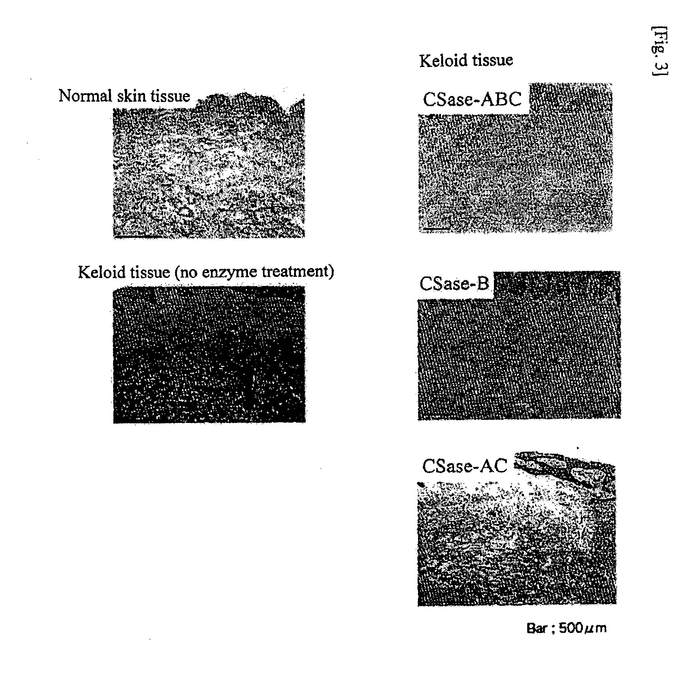

[Fig. 3] Fig. 3 are figures (photographs) showing the results of analyses on the accumulated CSs in keloid tissues. The upper left figure is a specimen of which a normal skin tissue section was stained with Alcian blue without an enzyme treatment. The lower left figure is a specimen of which a keloid tissue section was stained with Alcian blue without an enzyme treatment. The figures on the right are specimen photographs of which a keloid tissue section was stained with Alcian blue after a treatment with, from the top of the three figures, CSase-ABC, CSase-B, or CSase-AC.

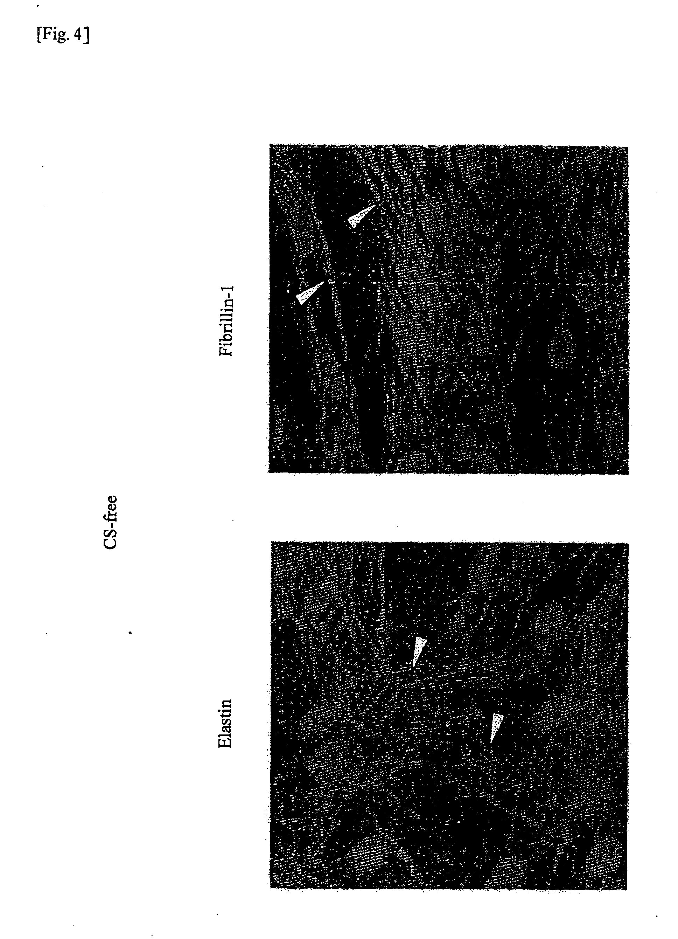

[Fig. 4] Fig. 4 are figures (photographs) showing the elastic fiber formation which were evaluated by *in vitro* assay for elastic fiber formation (elastogenesis assay). The left figure shows the staining result showing the localization of elastin fibers in the extracellular matrix, while the right figure shows the staining result showing the localization of fibrillin-1 in the extracellular matrix.

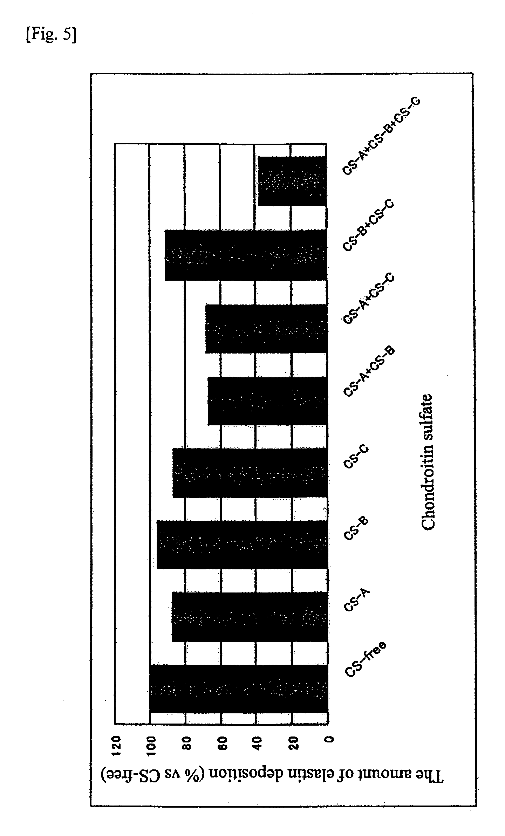

[Fig. 5] Fig.5 are a figure (graph) showing the inhibitory effects of various CSs (CS-A, CS-B or CS-C alone, and combinations of these CSs) on the elastic fiber formation, which were evaluated by *in vitro* assay for elastic fiber formation. After staining the elastin fibers deposited in the extracellular matrix, the areas of elastin deposition were converted into numerical values. The relative ratios of various CS-added groups in terms of the area of elastin deposition with respect to that of the CS-free group were expressed in a graph.

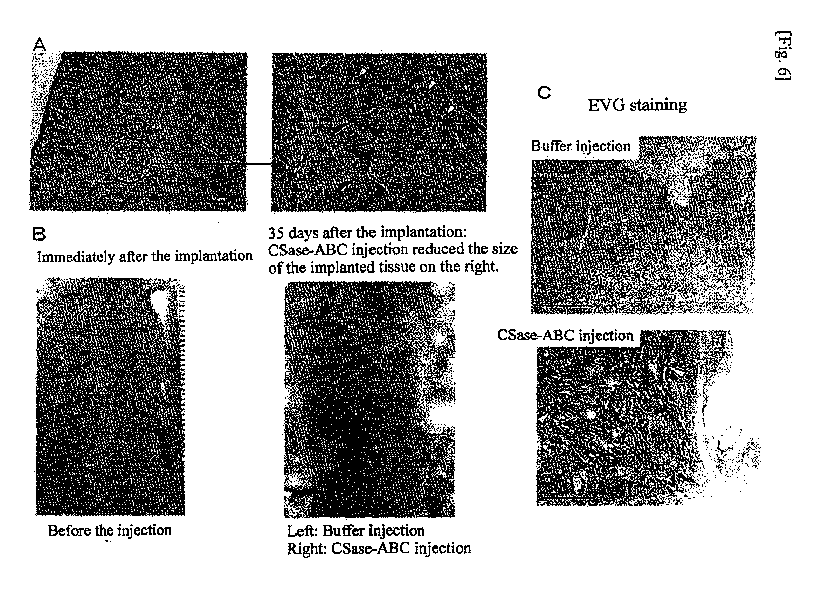

[Fig. 6] Fig. 6 are figures (photographs) showing the results of the therapeutic effects of CSase-ABC injection on the formation of elastic fibers *in vivo.* The two photographs of Fig. 6A are a photograph of the keloid lesion of a patient and a photograph of histopathological specimen thereof (prior to the implantation). The figure on the right is an enlargement of the part indicated with a circle in the figure on the left. The two figures (photographs) of Fig. 6B show the efficacy of CSase-ABC injected into the keloid tissues which were subcutaneously implanted on the back of a nude mouse at two sites. The left figure of Fig. 6B shows the implantation sites immediately after the implantation, and the right figure of Fig. 6B shows the implantation sites 35th day after implantation with the treatments of CSase-ABC or buffer. The two figures (photographs) of Fig. 6C were taken on the 35th day after the implantation and show the results under a microscope on the keloid tissues of the group injected with CSase-ABC or buffer. From the top, the figures (photographs) show the Elastica-van Gieson (EVG)-stained keloid tissue of the group injected with buffer and that of the group injected with CSase-ABC.

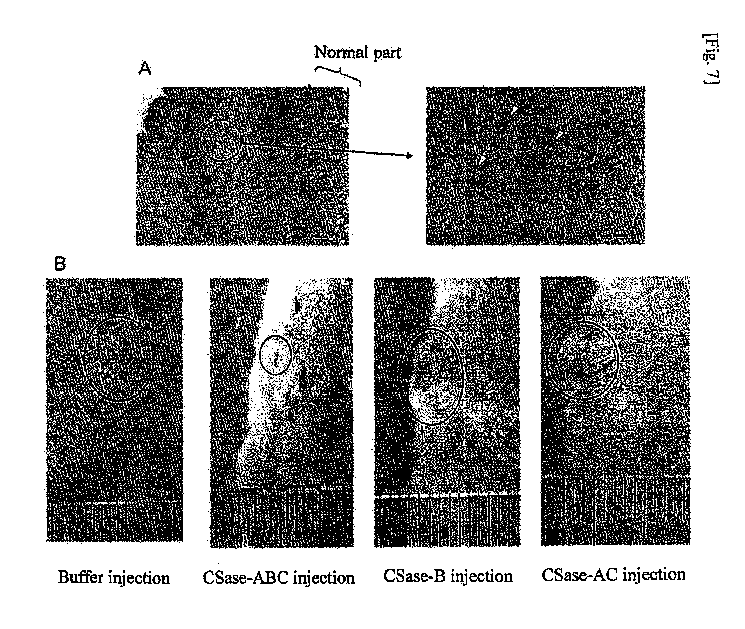

[Fig. 7] Fig. 7 are figures (photographs) showing the results of therapeutic effects of buffer injection, CSase-ABC injection, CSase-B injection and CSase-AC injection on the formation of elastic fibers *in vivo.* The two photographs of Fig. 7A show the histopathological specimen of the lesion portion of a patient (left) and an enlargement of the circled part (right) (prior to the implantation). Fig. 7B are figures (photographs) taken on the 35th day after the implantation, showing the observation results of therapeutic effects of buffer injection, CSase-ABC injection, CSase-B injection and CSase-AC injection, which injections were carried out after the subcutaneous implantation of the keloid tissues on the back of a nude mouse.

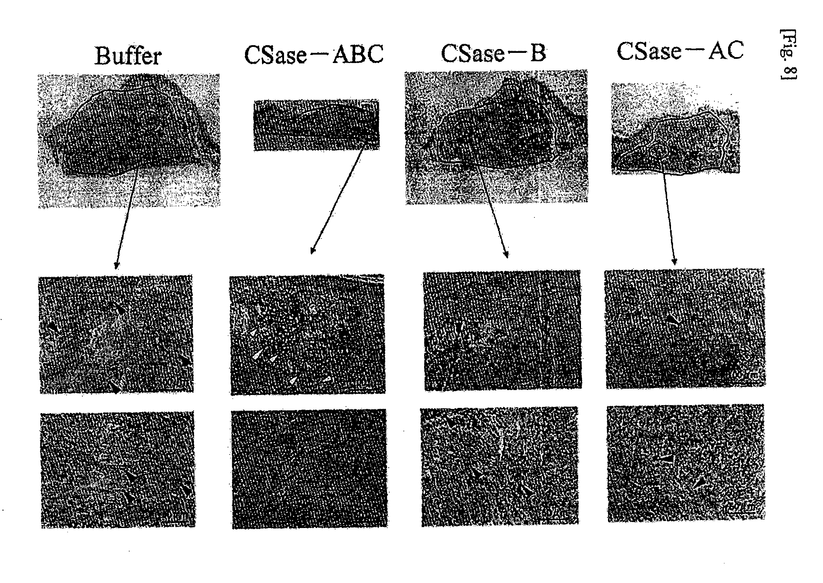

[Fig. 8] Fig. 8 are figures (photographs) taken on the 35th day after the implantation, showing the results of histological examinations as to the therapeutic effects of buffer injection, CSase-ABC injection, CSase-B injection and CSase-AC injection, which injections were carried out after the subcutaneous implantation of the keloid tissues on the back of a nude mouse. From the left, the photographs show the results of buffer injection, CSase-ABC injection, CSase-B injection and CSase-AC injection. The upper row are micrographs taken at a low magnification, capturing the areas of remaining implanted tissues. The middle row are micrographs taken at a medium magnification, capturing the condition of regeneration of elastic fiber structures, as well as the conditions of collagen fiber bundles and hyalinization thereof. The bottom row are micrographs taken at a high magnification, capturing the remaining keloid cells. The photographs in the upper and middle rows show the results of Elastica-van Gieson (EVG) staining. The photographs in the bottom row show the results of HE staining. The photographs of EVG staining in the middle row show the degree of elastic fiber regeneration in the tissues injected with buffer, CSase-ABC, CSase-B or CSase-AC. With photographs taken at different magnifications, the areas of the remaining implanted tissues, the degree of the regeneration of elastic fibers and the number of human keloid cells in the tissues after the theatments of buffer injection, CSase-ABC injection, CSase-B injection or CSase-AC injection were shown.

### DESCRIPTION OF EMBODIMENTS

The present invention will now be described in more detail.

### <1> The promoting agent according to the present invention

The elastic fiber formation promoting agent according to the present invention is an elastic fiber formation promoting agent containing an enzyme(s) which degrades CS-A, CS-B and CS-C (hereinafter, also referred to as "the promoting agent according to the present invention").

Herein, degradation of CS-A refers to an action to generate an unsaturated disaccharide-4-sulfate by cleaving the N-acetylhexosaminide bond in CS-A by elimination reaction. Degradation of CS-B refers to an action to generate an unsaturated disaccharide or tetrasaccharide by acting on CS-B, and degradation of CS-C refers to an action to generate unsaturated disaccharide (or tetrasaccharide)-6-sulfate by cleaving the N-acetylhexosaminide bond in CS-C by elimination reaction.

As the enzyme used in the promoting agent according to the present invention, specifically, CSase-ABC (derived from *Proteus vulgaris*; JP-A-1994(H6)-153947, T. Yamagata, H. Saito, O. Habuchi and S. Suzuki: J. Biol. Chem., 243, 1523(1968); and S. Suzuki, H. Saito, T. Yamagata, K. Anno, N. Seno, Y. Kawai and T. Furuhashi: J. Biol. Chem., 243, 1543(1968)) can be used. Further, as the CSase-ABC used in the present invention, those that are commercially available can be used. For instance, Chondroitinase ABC (catalog number: 100332), which is manufactured by Seikagaku Corporation, is exemplified.

Further, as the enzyme which can be used in Examples, in addition to the above CSase-ABC,
CSase-AC (derived from *Flavobacterium heparinum;* T. Yamagata, H. Saito, O. Habuchi, S. Suzuki, J. Biol. Chem., 243, 1523(1968)), CSase-ACII (derived from *Arthrobacter aurescens;* K. Hiyama and S. Okada, J. Biol.Chem.,250, 1824 (1975); and K. Hiyama and S. Okada, J. Biochem. (Tokyo), 80, 1201(1976)), CSase-ACIII (derived from *Flavobacterium* sp. Hp102; H. Miyazono, H. Kikuchi, K. Yoshida, K. Morikawa and K. Tokuyasu, J. Biochem., 61, 1023 (1989)), CSase-B (derived from *Flavobacterium heparinum;* Y. M. Michelacci and C. P.Dietrich, Biochem. Biophys. Res. Commun.,56, 973(1974), Y. M. Michelacci and C. P. Dietrich, Biochem. J., 151, 121(1975), and K. Maeyama, A. Tawada, A. Ueno and K. Yoshida, J. Biochem., 57, 1189 (1985)) and the like are known. Any one of these chondroitinases can also be used.

Further, in the above CSase-ABC used in the present invention, it is known that, in addition to the major component of CSase-ABC (hereinafter, also referred to as "lyase I"), so-called "chondroitin sulfate lyase II" (JP-A-1998(H10)-262660; hereinafter, referred to as "lyase II") is also contained. As the enzyme used in the present invention, a form which includes the lyase II can be used, and a highly purified fraction containing only a fraction of each lyase can also be used. Among these, it is preferred to use a highly purified fraction containing only lyase I.

For the method of obtaining the above-described high-purity CSase-ABC fraction (lyase I fraction) and lyase II fraction, for example, JP-A-2002-335968 and JP-A-1998(H10)-262660 can be referred to.

Further, the expression "derived from" as to CSase-ABC used herein, means that the origin thereof is the gene encoding the CSase in a living organism which intrinsically carries the gene. For example, CSase-ABC derived from *Proteus vulgaris* refers to a CSase-ABC which is produced by the gene intrinsically carried by *Proteus vulgaris.* Accordingly, the chondroitinase ABC derived from *Proteus vulgaris* includes not only those produced by *Proteus vulgaris* itself, but also those that were produced by other cells using the CSase-ABC gene obtained from *Proteus vulgaris,* and the like. Furthermore, the chondroitinase ABC derived from *Proteus vulgaris* includes recombinant variants of a CSase-ABC or the like, which were produced by using a variant CSase-ABC gene made from the above-described gene.

The enzyme used in the present invention is preferably one which was purified to such an extent that it can be used as a pharmaceutical and does not substantially contain a substance that is not permitted to be included in a pharmaceutical. For example, it is preferred that the enzyme be a purified CSase-ABC having an enzyme activity of not less than 100 U/mg protein. It is especially preferred to use a purified CSase-ABC having an enzyme activity of not less than 100 U/mg protein, which does not substantially contain an endotoxin and whose nucleic acid content and protease content are not higher than the detection limit.

Herein, 1 U (unit) of a CSase is the amount of enzyme which catalyzes the formation of the product from CS at a rate of 1 micromole per minute at an optimum pH and at around an optimum temperature. For the record, the definition of 1 U of various CSases are shown in the following.

The 1 U of CSase-ABC is defined as the amount of enzyme which catalyzed the formation of unsaturated disaccharide from CS at a rate of 1 micromole per minute at pH 8.0 and 37°C. Further, 1 U of CSase-AC (derived from *Flavobacterium heparinum*) is defmed as the amount of enzyme which catalyzed the formation of unsaturated disaccharide from CS at a rate of 1 micromole per minute at pH 7.3 and 37°C.

Further, 1 U of CSase-B (derived from *Flavobacterium heparinum*) is defined as the amount of enzyme which generates a UV-absorbing substance corresponding to Δ4-hexuronic acid residue from CS-B at a rate of 1 micromole per minute at pH 8.0 and 37°C.

The enzyme activity of the enzyme used in the present invention and Examples can be quantified by measuring the amount of generated unsaturated disaccharide under the above-described optimum condition of each enzyme and comparing it to the amount generated by 1 U of the enzyme.

For example, by using a chondroitinase ABC having an enzyme activity of not less than 100 U/mg protein, provided is a highly safe and effective pharmaceutical, which does not affect the surrounding tissues when administered into a living body as a injectable pharmaceutical and can appropriately degrades CS of proteoglycans at a target site (for example, a keloid or hypertrophic scar). Such a CSase-ABC can be obtained by, for example, the method described in JP-A-1994(H6)-153947. A commercially available CSase-ABC can also be used.

The promoting agent according to the present invention can also be used as a reagent of an experiment.

Further, there is also described the concept of an elastic fiber regenerating agent containing an enzyme which degrades CS-A, CS-Band CS-C (hereinafter, also referred to as "the regenerating agent according to the present invention").

It is preferred to employ a CSase-ABC as the enzyme used in the regenerating agent. Among the CSase-ABCs, those derived from *Proteus vulgaris* are preferred, and it is very preferred to use a highly purified fraction containing only the lyase I.

As described in the above, in keloid tissues, elastin is not deposited and/or cross-linked to fibrillin-1 protein; therefore keloid tissues are deficient in the elastic fiber formation. Here, by administering the regenerating agent according to the present invention, the association between elastin and fibrillin can be induced to regenerate elastic fibers. The explanation regarding "enzyme which degrades CS-A, CS-B and CS-C" and the like used in the regenerating agent according to the present invention, as well as the preferred method of administration thereof, the number of the administrations and the like are the same as those described for the therapeutic agent according to the present invention.

### <2> The therapeutic agent according to the present invention

The therapeutic agent according to the present invention is a radical therapeutic agent for keloids and/or hypertrophic scars, containing an enzyme which degrades CS-A, CS-B and CS-C (hereinafter, also referred to as "the therapeutic agent according to the present invention").

The therapeutic agent according to the present invention may contain only an enzyme which degrades CS-A, CS-B and CS-C as the effective component. Further, the therapeutic agent according to the present invention may be blended with yet another medicinal component along with the enzyme. Here, the medicinal component is not particularly restricted, as long as one of the substances does not inhibit the intrinsic action of the other substance by being blended with the enzyme or by administered in combination.

Further, the administration route of the therapeutic agent according to the present invention is not particularly restricted. The effective component according to the present invention can be dissolved into, for example, water, buffer solution or physiological saline, and can be injected, for example, subcutaneously, intradermally or intramuscularly.

Further, the therapeutic agent according to the present invention may be used in combination with a local anesthetic or the like.

In the present invention, a treatment encompasses not only those ex-post treatments in a conventional meaning, which are carried out for a patient after he/she is affected by the target disorder, but also preventive measures which are carried out before the fact in order to prevent the development or recurrence of a keloid and/or hypertrophic scar.

Further, in the present invention, a radical treatment does not refer to palliative measures which simply reduce the size of the lesion site or the like, but rather means to radically and completely cure the lesion site by allowing it to recover to a normal tissue.

As seen from the Reference Examples described later, when keloid cells are cultured in a culture system in which CS-A, CS-B and/or CS-C (co-)exist(s), the deposition of elastins to the extracellular matrix, that is, the association thereof with fibrillin, is inhibited. Further, as seen from the Examples, by administering an enzyme which degrades CS-A, CS-B and CS-C to the keloid tissue, the regeneration of elastic fibers takes place by the association between elastin and fibrillin, thereby enabling a radical treatment of the keloid and/or hypertrophic scar.

The therapeutic agent according to the present invention contains an enzyme which degrades CS-A, CS-B and CS-C at an effective amount to treat a keloid and/or hypertrophic scar by promoting the association between elastin and fibrillin, that is, by allowing elastic fibers to regenerate.

The phrase "an effective amount" used herein refers to an amount which is effective to promote the regeneration of elastic fibers in a keloid and/or hypertrophic scar in order to improve, normalize and prevent the recurrence of the keloid and/or hypertrophic scar. This amount varies depending on the symptom(s) of the patient, the volume of the lesion portion, the patient's age and the like. The amount is not particularly restricted as long as the amount is effective to promote the formation of elastic fibers in keloids and/or hypertrophic scars, and to improve the condition of keloid and/or hypertrophic scar, and to prevent the reoccurence of keloid and/or hypertorophic scar, and examples of such amount include, for example, in a single dose of the therapeutic agent according to the present invention per 5 mm³ of lesion portion, not less than 0.01 U, not less than 0.02 U, not less than 0.05 U, not less than 0.1 U, not less than 0.5 U, not less than 1 U, not less than 2 U and not less than 4 U. More specifically, the ranges of 0.01 to 5 U, 0.01 to 4 U, 0.01 to 2 U, 0.01 to 1 U, 0.01 to 0.5 U, 0.01 to 0.1 U, 0.01 to 0.05 U, 0.01 to 0.02 U, 0.02 to 5 U, 0.02 to 4 U, 0.02 to 2 U, 0.02 to 1 U, 0.02 to 0.5 U, 0.02 to 0.1 U, 0.02 to 0.05 U, 0.05 to 5 U, 0.05 to 2 U, 0.05 to 1 U, 0.05 to 0.5 U, 0.05 to 0.1 U, 0.1 to 5 U, 0.1 to 4 U, 0.1 to 2 U, 0.1 to 1 U, 0.1 to 0.5 U, 0.5 to 5 U, 0.5 to 4 U, 0.5 to 1 U, 1 to 5 U, 1 to 4 U, 1 to 2 U, 2 to 5 U, 2 to 4 U and 4 to 5 U can be exemplified.

Further, the number of administrations of the therapeutic agent according to the present invention may be once a day, and the therapeutic agent according to the present invention may be administered 2 to 4 times a day or more times a day. Such administration can be given every day as required or at intervals of appropriate days over a necessary period of time.

The subject to which the therapeutic agent according to the present invention is applied is not particularly restricted as long as it is a keloid and/or hypertrophic scar. The therapeutic agent according to the present invention can be broadly applied to, for example, true keloids, cicatrical keloids, hypertrophic scars and mature scars (examples thereof include acne scars).

### EXAMPLES

The present invention will now be described in more detail by way of examples thereof; however, the present invention is not restricted thereto.

### [Reference Example 1] Observation of keloid lesion portion and normal skin part

As a tissue material, a human tissue sample containing a keloid lesion and normal skin part was extirpated from a keloid patient. The sample was fixed in 4% paraformaldehyde at 4°C for 24 hours and subsequently embedded in paraffin to prepare a paraffin block from which a 3-µm paraffin section was prepared. After deparaffinization, hematoxylin and eosin (HE) staining and Elastica-van Gieson (EVG) staining were performed on the thus obtained paraffin section to prepare a specimen. The keloid tissue and normal skin tissue were observed under a microscope.

The results were shown in Fig. 1. The part which was indicated with a line is the keloid lesion portion and the adjacent part is the normal skin part. By EVG staining, the elastic fiber formation (indicated by arrows), which are stained in black, can be confirmed in the normal skin part other than keloid lesion portion. In contrast, hyalinization is observed in the greater part of the keloid lesion portion, and it can be confirmed that the keloid lesion portion is deficient in the formation of the elastic fiber.

### [Reference Example 2] mRNA expression of the elastic fiber constituents in the lesion tissue of keloid and normal skin tissue

As tissue materials, human samples were extirpated from the keloid lesion tissues (4 individuals) and normal skin tissues (3 individuals). From these keloid tissues and normal skin tissues, total RNAs were extracted using RNeasy Plus kit (manufactured by QIAGEN). From 1 µg of the thus obtained total RNAs, cDNAs were synthesized using Advantage RT for PCR kit (manufactured by Becton, Dickinson and Company of Japan). For seven types of proteins that are the constituents of elastic fibers, the mRNA expressions thereof were examined by RT-PCR. The primers used in this PCR are shown in Table 1.

The specific sequences were amplified by performing PCR reactions using Blend Taq-plus (registered trademark) (manufactured by Toyobo Co. Ltd.), and the thus obtained PCR products were verified by electrophoresis. The PCR reactions were performed at the following conditions: denaturation at 94°C for 30 seconds, annealing at 58°C for 30 seconds and extension at 72°C for 1 minute. This cycle was repeated 30 times for DANCE, MFAP-2 and GAPDH, and 35 times for tropoelastin, fibrillin-1, fibrin-1 and EMILIN.

**[Table 1]**

| Target | | DNA sequence | Predicted size (bp) |
|---|---|---|---|
| Tropoelastin | F | 5'AAGCAGCAGCAAAGTTCG3'(SEQ ID NO:1) | |
| | R | 5'ACCTGGGACAACTGGAATCC3'(SEQ ID NO:2) | 287 |
| Fibrillin-1 | F | 5'GTGAGATCAACATCAATGGAGC3'(SEQ ID NO:3) | |
| | R | 5'TTACACACTCCTGGGAACACTTC3'(SEQ ID NO:4) | 180 |
| Fibrin-1 | F | 5'GATGTCCTCCTGGAGGCCTGCTGTG3'(SEQ ID NO:5) | |
| | R | 5'TTGGGTCGGCAGCGGAAGGATCCCAG3'(SEQ ID NO:6) | 783 |
| DANCE | F | 5'CGGCACATACTTCTGCTCCT3'(SEQ ID NO:7) | |
| | R | 5'TCAGAATGGGTACTGCGACA3'(SEQ ID NO:8) | 549 |
| EMILIN | F | 5'ATTATGACCAGAGACAGGC3'(SEQ ID NO:9) | |
| | R | 5'CCGAGTGCGCCAGCTGCCCC3'(SEQ ID NO:10) | 290 |
| MFAP-2 | F | 5'ATGAGAGCTGCCTACCTCTTC3'(SEQ ID NO:11) | |
| | R | 5'CTAGCAGCTCCCACAGCTCCT3'(SEQ ID NO:12) | 551 |
| GAPDH | F | 5'TGGTATCGTGGAAGGACTCATGAC3'(SEQ ID NO:13) | |
| | R | 5'ATGCCAGTGAGCTTCCCGTTCAGC3'(SEQ ID NO:14) | 189 |

The results were shown in Fig. 2A. Reference Example 1 indicated that the elastic fiber formation was not confirmed in the keloid lesion portion. In the present test, it was shown that mRNAs of the constituents of elastic fibers, such as elastin which is the major component of elastic fibers, fibrillin-1 and the like, were expressed in the keloid tissue at a level comparable to that of the normal tissue. This indicates that the deficiency in the elastic fiber formation in the keloid tissue is not the result of a reduction in the production of elastin, fibrillin-1 and the like.

### [Reference Example 3] Expressions of the elastin and fibrillin-1 proteins in the keloid tissues

Reference Example 2 indicated that mRNAs of elastin and fibrillin-1 were expressed at a normal level in the keloid tissues. Subsequently, the expressions of elastin and fibrillin-1 at the protein level, as well as the localization thereof in the extracellular matrix, were examined by immunohistochemical staining. The procedures thereof were as follows.

Elastin staining: A human keloid tissue was extirpated and fixed in 4% paraformaldehyde at 4°C for 24 hours. The thus fixed tissue was then embedded in paraffin and a 6-µm section was prepared therefrom. After deparaffinization, immunohistochemical staining was performed using LSAB/HRP kit (manufactured by Dako Japan, Inc.). As the primary antibody, an anti-elastin antibody (1:100; manufactured by Elastin Products Company, Inc., PR533) was used.

Fibrillin-1 staining: A human keloid tissue was extirpated, which was then immediately embedded in OCT compound and frozen. A 10-µm frozen section was prepared. The thus obtained section was blocked with Block Ace and was subsequently allowed to react with an anti-fibrillin-1 antibody (1:200; manufactured by NeoMarkers Inc.). As the secondary antibody, Alexa Fluor 546 goat anti-rabbit IgG antibody (1:800; manufactured by Molecular Probes, Inc.) was used. Additionally, nuclear staining was performed on the cells using Hoechst (manufactured by Sigma).

The results were shown in Fig. 2B. The parts that were stained by Hoechst staining indicate the localization of cell nuclei, that is, the localization of cells, and present in spaces between the cells is the extracellular matrix. In elastin staining, although the expressions of elastin (indicated by arrows) can be confirmed within the cells, no fibrous stained image was observed in the extracellular matrix (as an exemplification, an area of the extracellular matrix was indicated with *). In fibrillin-1 staining, fibrous stained images were observed in the extracellular matrix in the same manner as in the normal skin.

These results indicate that, while both elastin and fibrillin-1 are being produced in the cells of the lesion portion of the keloid patient, deposition of elastin to fibrillin-1 is not confirmed in the extracellular matrix, and that the elastic fiber formation is deficient. It is believed that, although the produced elastins are excreted into the extracellular matrix, they are metabolized and eliminated from the keloid tissue because they do not associate with and are deposited to fibrillin-1 as elastic fibers.

### [Reference Example 4] Analyses of CSs accumulated in the keloid tissue

The amount of the accumulated CSs was compared between the keloid tissue and normal skin. Additionally, the types of the CSs accumulated in the keloid tissue were also examined.

The analyses were carried out by Alcian blue staining (pH 2.5) after deparaffinization of a keloid tissue section (6µm) and normal skin tissue section (6µm) that were obtained in the same manner as in Reference Example 3, which were subsequently left untreated or subjected to various CSase treatments. Alcian blue is a dye which stains GAGs in a tissue. Each of CSase-ABC (derived from *Proteus vulgaris,* manufactured by Seikagaku Corporation), CSase-B (derived from *Flavobacterium heparinum,* manufactured by Seikagaku Corporation) and CSase-AC (derived from *Flavobacterium heparinum,* manufactured by Seikagaku Corporation) was dissolved into 0.1 mol Tris-HCl buffer to a concentration of 1 mU / 1 µl, and after treating the tissue sections with the prepared enzyme solution at 37°C for 2 hours, they were stained with Alcian blue solution (pH 2.5).

The results were shown in Fig. 3. The two figures on the left are the photographs of the keloid tissue section without CSase treatment (the bottom figure) and the normal skin tissue section with no treatment (the top figure), which were stained with Alcian blue. It can be seen, in comparison to the normal skin tissue, that the staining property of the keloid tissue is stronger and that GAGs were accumulated in the extracellular matrix of the keloid lesion portion. Meanwhile, the three photographs on the right are those of the keloid tissue sections which were stained with Alcian blue after the treatment with CSase-ABC, CSase-B or CSase-AC. Compared to the keloid tissue (no enzyme treatment) of the photograph in the bottom left , the staining properties for Alcian blue were reduced in all of the enzyme-treated keloid tissues. As for the keloid tissue treated with CSase-ABC, it was confirmed that the staining property was reduced to a level comparable to that of the normal tissue.

### [Reference Example 5] The effects of CSs in the elastic fiber regeneration

The results of Reference Example 4 suggested that the excessive accumulation of CS causes the deficiency in the elastic fiber formation (inhibition of elastin deposition to fibrillin) in the keloid tissue. In view of this, in an *in vitro* culture system (elastogenesis assay), keloid cells derived from a patient were artificially treated with only one of CS-A, CS-B and CS-C, or with CSs in combination, and it was examined which combination most prominently causes the deficiency in the elastic fiber formation (inhibition of elastin deposition in the extracellular matrix).

The culture plate which is 13mm in diameter is inseted into a 24-well cell-culture plate (manufactured by Iwaki), and keloid cells collected from a human keloid tissue by an explant method were inoculated into the culture plate at a concentration of 1 × 10⁴ cells/well and cultured in DMEM medium (manufactured by Gibco, Inc.). The CS-added groups were set up with addition of only one of CS-A, CS-B and CS-C, or two or three of CS-A, CS-B and CS-C in combination. One ml of medium containing one of the above 400 µg/ml CS-A, CS-B and CS-C, or two or three of 400 µg/ml CS-A, CS-B and CS-C in combination was added to the keloid cells at an interval of 2 days. Further, as a control, the keloid cells were cultured without an addition of CS(s) (CS-free group). On day 9 of culture, the culture plate which has the cells and the extracellular matrix around the cells were taken out from the 24-well cell-culture plate and fixed with 100% methanol and then with 2% BSA. Thereafter, the thus fixed cells and the matrix were allowed to react with the primary antibodies, anti-elastin antibody (1:100; manufactured by Elastin Products Company, Inc., PR533) and anti-fibrillin-1 antibody (1:200; manufactured by Elastin Products Company, Inc., PR217). Alexa Fluor 546 goat anti-rabbit IgG antibody (1:200; manufactured by Molecular Probes, Inc.) was used as the secondary antibody, and nuclear staining was performed on the cells using Hoechst (manufactured by Sigma). Subsequently, the thus stained cells and the matrix were observed under a confocal laser scanning microscope system (manufactured by Nikon Corporation, Digital Eclipse C1si), and images of the stained cells and the matrix were obtained. Then, the thus obtained images of elastin staining were analyzed using an image analysis software (Image pro). Gray-scaling and image inversion were performed on the images. The image range was selected while comparing with the images of nuclear staining, so that the stained areas not containing the regions of stained nuclei could be extracted. The areas of the elastin-stained parts in the matrix were measured. The thus obtained values were indicated in relative %, taking the value for the CS-free group as 100%.

The photographs of elastin staining and fibrillin-1 staining of the CS-free group were shown in Fig. 4. The stained elliptical parts indicate the parts of cell nuclei stained by Hoechst staining, that is, the localization of cells, while the spaces between the cells indicate the parts of extracellular matrix. For the CS-free group, elastin deposition in the extracellular matrix (photograph on the left: indicated by white arrows) and the fiber structures of fibrillin-1 (photograph on the right: indicated by white arrows) were confirmed. The images of all the CS-added groups were analyzed using the image analysis software, and the results thereof were shown in a graph and table (Fig. 5 and Table 2). In those groups of which the cells were added with only one of CS-A, CS-B and CS-C, or with two of CS-A, CS-B and CS-C in combination, a slightly more inhibitory effect on the deposition of fibrous elastins was found in the extracellular matrix compared to the CS-free group, and the strongest inhibitory effect on the deposition of fibrous elastins was found in the group of which the cells were added with three types of CSs, CS-A, CS-B and CS-C, simultaneously. That is, it indicated that the most prominent inhibitory action of the elastic fiber formation is induced when these three types of CSs were allowed to coexist.

**[Table 2] The amount of elastin deposition to the extracellular matrix at the time of addition with various CSs (%)**

| | CS-free | CS-A | CS-B | CS-C | | | | CS-A |
|---|---|---|---|---|---|---|---|---|
| | | | | | CS-A | CS-A | CS-B | CS-B |
| | | | | | CS-B | CS-C | CS-C | CS-C |
| % vs CS-free | 100 | 87.0 | 95.9 | 86.7 | 66.3 | 67.4 | 91.0 | 37.6 |

### [Example 1] The effects of CSase-ABC on the elastic fiber formation (regeneration) in implanted keloid lesion tissue

The results of Reference Example 4 suggested that the CSs accumulated in the keloid tissues are most effectively degraded by CSase-ABC. Further, the results of Reference Example 5 suggested that the strongest inhibition of the elastic fiber formation is induced when all of CS-A, CS-B and CS-C coexist. In view of these, a CSase-ABC capable of degrading all three types of CSs was selected and the therapeutic effects thereof were investigated *in vivo* (in an implanted keloid lesion tissue). That is, the effects of the selected enzyme on the elastic fiber formation (regeneration) and the size of the keloid tissue were investigated.

Fig. 6A shows the photographs of the histopathological specimen of the tissue section taken from a keloid patient (the clinical symptoms were severe) which was used in the implantation. The figure on the right is an enlargement of the part indicated with a circle in the figure on the left. The large and bright fibroblasts are keloid cells, which are many (indicated by white arrows), and intricate hyalinized collagen fiber bundles are prominently noticeable (indicated by black arrows). The tissue is deficient in elastic fibers. In the figures, features of a normal skin cannot be found in the keloid tissue. From the lesion tissue shown in Fig. 6A, keloid tissues of 5 mm square were taken and implanted into the back of an immunodeficient mouse (c57 balb nu/nu 6-week-old male (manufactured by Japan SLC, Inc.)) at two sites. Upon confirmation of the engraftment, 8 days after the implantation and 18 days after the implantation, 10 µl of 50 mU / 10 µl chondroitinase ABC (derived from *Proteus vulgaris,* manufactured by Seikagaku Corporation) dissolved in 0.1M Tris buffer was injected to one of the implanted tissue sections (the site of the implantation on the right side). 10 µl of 0.1M Tris buffer was injected topically to the other implanted tissue section (the site of the implantation on the left side) as a control. The sizes of the tissue sections were visually observed and photographs thereof were taken 35 days after the implantation.

In the same manner as described in the above, keloid tissues of 5 mm square were taken from the same lesion tissue, and they were implanted into the back of an immunodeficient mouse. Upon confirmation of the engraftment, 7 days, 14 days and 21 days after the implantation, 10 µl of 50 mU / 10 µl chondroitinase ABC dissolved in 0.1M Tris buffer was injected to one of the implanted tissues. 10 µl of 0.1M Tris buffer was injected topically to the other implanted tissues as a control. The implanted tissues were taken from the mice 35 days after the implantation (6 weeks after the start of the experiment) and they were subjected to histological analyses. After fixing the tissue in 4% paraformaldehyde at 4°C for 24 hours, paraffin blocks were prepared. Therefrom, a 3-µm section was prepared from each paraffin block, and after deparaffinization, Elastica-van Gieson (EVG) staining was performed on the section. Thereafter, the appearance of skin tissue of the implantation site on the section were observed under a microscope.

Fig. 6B shows the photographs of the skin tissues of the implantation sites immediately after the implantation of the keloid tissue into the immunodeficient mouse and 4 weeks after the implantation.

The photograph on the left shows the condition immediately after the implantation of the keloid tissue section, while the photograph on the right was taken 35 days after the implantation. In the photograph on the right, the implantation site in the left is the keloid tissue injected with the buffer (control), while the implantation in the right is the keloid tissue injected with CSase-ABC. As can be seen from the photograph, the keloid tissue in the right reduced considerably by the CSase-ABC injection, compared to the implanted tissue injected with the buffer.

Fig. 6C shows the photographs of the EVG staining on the keloid tissue sections prepared from the implanted tissure at 35 days after the implantation, which were injected buffer or CSase-ABC.

According to the results, the elastic fiber formation was not observed at all in the tissue of the buffer-injected group with most parts of the tissue still hyalinized, while the formation of elastic fibers, which were stained in black (indicated by black arrows), could be observed over a large area in the tissue of the CSase-ABC injected group, thereby confirming that the elastic fiber formations were being regenerated. Furthermore, the condition of the collagen fiber bundles, which were stained in red (indicated by white arrows), became a condition similar to that of normal skin tissue, and hyalinization disappeared.

From the above, it was confirmed that the CSase-ABC injection induced the regeneration of elastic fibers, disappearance of intricate collagen fiber bundles and hyalinization, as well as considerable reduction in the volume of the keloid tissue, thereby demonstrating the initial idea of the inventors that CSase-ABC injection can be a radical therapeutic agent for keloids, having regeneration of elastic fibers as its mechanism.

### [Example 2] The comparison of therapeutic effects of CSase-ABC, CSase-B and CSase-AC

The therapeutic effects of CSase-ABC, CSase-B and CSase-AC were compared in the same manner as in Example 1. Each of CSase-ABC, CSase-B and CSase-AC was dissolved into 0.1 mol Tris-HCl buffer to a concentration of 50 mU / 10 µl, and the thus obtained solutions were each injected into the implanted tissue at an amount of 10 µl. Fig. 7A shows the photographs of the histopathological specimen of the lesion portion of a keloid patient (the clinical symptoms were moderate) from who the implanted sections were collected. The figure on the right is an enlargement of the part indicated with a circle in the figure on the left. The number of the keloid cells (indicated by white arrows) is larger; however, the degree of hyalinization of collagens is less compared to Example 1 (Fig. 6A). The tissue is deficient in elastic fibers and normal region is not found in the figure on the right. Fig. 7B shows the photographs of one side of the back of the mice to which the keloid tissues derived from the patient were implanted. To the tissues, from the left in Fig.7B, buffer, CSase-ABC, CSase-B, or CSase-AC was injected once a week for a total of three times after engraftment. The photographs were taken on the 35th day after the implantation. For the tissue injected with CSase-ABC, a considerable reduction in the size of the implanted tissue was observed, and the keloid tissue was flattened to a degree such that the keloid tissue cannot be distinguished from the skin of the mouse. A slight reduction in the size of the implanted tissue section was observed for the tissue injected with CSase-B, while a reduction in the size of the implanted tissue was hardly observed for the tissue injected with CSase-AC.

The same histological examination as in Example 1 was carried out in order to confirm whether the image of the implanted tissue after the injection of CSase-ABC had reached the histopathological image same as that of a normal tissue, that is, whether regeneration of elastic fibers and disappearance of intricate collagen bundles and hyalinization could be seen. For comparison, the same histological examination was carried out for the mice injected with buffer, CSase-B or CSase-AC (Fig. 8). The areas of the remaining keloid tissue were circled in the upper row. In order to calculate the relative values of the remaining human keloid tissue areas of the cases where CSase-B, CSase-AC or CSase-ABC was injected with respect to that of the case in which the buffer was injected, the areas of the remaining keloid tissue (the areas inside the circles) were measured using Image-Pro Express J5.1. Compared to the case of buffer injection, a slight reduction in the area of the remaining human keloid tissue was observed for the case of CSase-B injection, while a reduction in the area by about half in the area were observed for the case of CSase-AC injection and a considerable reduction in the area were observed for the case of CSase-ABC injection. The result for the case of CSase-ABC injection was consistent with that of visual inspection of Fig. 7B. In order to show the degree of elastic fiber regeneration and the condition of hyalinization, the middle row of Fig. 8 shows the tissue photographs, which are enlargements of each of the circled part in the upper row.

A high degree of hyalinization and many intricate collagen fibers are observed (indicated by black arrows) for the mice injected with buffer, CSase-B or CSase-AC. However, for the mouse with CSase-ABC injection, hyalinization and intricate collagen fibers are not observed and definite regenerations of elastic fibers (indicated by white arrows) are observed. It is speculated that the reason why the regeneration of elastic fibers was not observed over the entire tissue compared to the results of the mouse injected with CSase-ABC in Example 1 is because the keloid tissue started to be assimilated into the tissue of the nude mouse after being normalized. It has been already known that the formation of elastic fibers is poor in the content in the skin of a nude mouse. Mice injected with CSase-B or CSase-AC were not observed with a definite elastic fiber formation. The structures which are seen in a deep color in the center of the photograph of the tissue of the mouse injected with CSase-B in the middle row are not elastic fibers, but intricate collagen bundles (stained in red). In the bottom row, the photographs were further enlarged to show the condition of the keloid cells (HE staining). Those cells, which are larger than a normal human fibroblast and whose nucleus is enlarged and brightly stained, are keloid cells (indicated by arrows). In the tissues injected with buffer, CSase-B, or CSase-AC, many keloid cells were observed; however, keloid cells were eliminated in the tissue injected with CSase-ABC (all of the cells were normal fibroblasts). The number of the keloid cells in one field of view was counted three times to calculate the average. Taking the case of buffer injection as 100%, the relative values of each case were indicated in %. The above-described results were summarized in Table 3.

**[Table 3]**

| Administered substance | Relative area of remaining human keloid tissue (% vs Buffer) | Elastic fiber formation | Hyalinization | Intricate collagen fiber bundles | The number of keloid cells (% vs Buffer) |
|---|---|---|---|---|---|
| Buffer | 100 | Absent | Present | Present | 100 |
| CSase-AC | 52 | Absent | Present | Present | 91 |
| CSase-B | 78 | Absent | Present | Present | 53 |
| CSase-ABC | 26 | Close to normal | Absent | Absent | 0 |
| Normal region | - | Normal | Absent | Absent | - |

As seen from the results of the CSase-AC injection and CSase-B injection in Table 3, it was found that a reduction in the size of the keloid tissue was not directly related to a reduction in the number of human fibroblasts (keloid cells). Furthermore, in the case of CSase-ABC injection, as the condition of the tissue attained the regeneration of elastic fibers, disappearance of intricate collagen bundles and hyalinization, and disappearance of keloid cells, it was confirmed that the therapeutic effects of CSase-ABC are exceptionally superior to those of other CSases.

### INDUSTRIAL APPLICABILITY

By the present invention, an elastic fiber formation promoting agent containing an enzyme which degrades CS-A, CS-B and CS-C, as well as a radical therapeutic agent for keloids and/or hypertrophic scars containing an enzyme which degrades CS-A, CS-B and CS-C, are provided. The above agents have an action to promote the regeneration of elastic fibers in keloids and/or hypertrophic scars, normalization of collagen fiber bundles and normalization of lesion tissues; therefore, these agents lead to a radical treatment. By these agents, keloids and/or hypertrophic scars can be completely treated without any possibility of recurrence thereof which is frequent in a conventional treatment with a steroid agent.

The therapeutic agent according to the present invention can, at a low dosage, completely treat keloid and hypertrophic scar, on which sufficient clinical effects were not attained by a conventional treating method or therapeutic agent. Furthermore, it can be widely used as a therapeutic agent which does not produce a severe side effect.

## Claims

1. *In vitro* use of chondroitinase ABC for promoting elastic fiber formation.

2. The *in vitro* use according to claim 1, **characterized in that** said chondroitinase ABC is derived from *Proteus vulgaris.*

3. Chondroitinase ABC for use in the radical treatment of keloids and/or hypertrophic scars.

4. The chondroitinase ABC for use according to claim 3, **characterized in that** an association between elastin and fibrillin is promoted by said enzyme.

5. The chondroitinase ABC for use according to claim 3, **characterized in that** the chondroitinase ABC is derived from *Proteus vulgaris.*

## Patentansprüche

1. In-vitro-Verwendung von Chondroitinase ABC zur Förderung der Bildung einer elastischen Faser.

2. Die In-vitro-Verwendung nach Anspruch 1, **dadurch gekennzeichnet, dass** die Chondroitinase ABC aus *Proteus vulgaris* entstammt.

3. Chondroitinase ABC zur Verwendung bei der Radikal-Behandlung von Keloiden und/oder hypertrophen Narben.

4. Die Chondroitinase ABC zur Verwendung nach Anspruch 3, **dadurch gekennzeichnet, dass** eine Verbindung zwischen Elastin und Fibrillin durch das Enzym gefördert ist.

5. Die Chondroitinase ABC zur Verwendung nach Anspruch 3, **dadurch gekennzeichnet, dass** die Chondroitinase ABC aus *Proteus vulgaris* entstammt.

## Revendications

1. Utilisation in vitro de chondroitinase ABC pour stimuler la formation de fibres élastiques.

2. Utilisation in vitro conforme à la revendication 1,
**caractérisée en ce que**
la chondroitinase ABC est dérivée de *Proteus vulgaris.*

3. Chondroitinase ABC destinée à être utilisée pour le traitement radicalaire de cicatrices chéloïdes et/ou hypertrophiques.

4. Chondroitinase ABC destinée à être utilisée conformément à la revendication 3,
**caractérisé en ce qu'**
une association entre l'élastine et la fibrilline est stimulée par cette enzyme.

5. Chondroitinase ABC destinée à être utilisée conformément à la revendication 3,
**caractérisé en ce que**
la chondroitinase ABC est dérivée de *Proteus _{U}ulgaris.*


### SEQUENCE LISTING

<110> Shigehiko SUZUKI
<120> An agent for radical treating keloid and hyperplastic scar
<130> F200805C8358
<150> JP2007-317294
   <151> 2007-12-07
<160>14
<210>1
   <211>18
   <212>DNA
   <213>Artificial Sequence
<220>
   <223>tropoelastin forward primer
<400>1
   aagcagcagc aaagttcg 18
<210>2
   <211>20
   <212>DNA
   <213>Artificial Sequence
<220>
   <223>tropoelastin reverse primer
<400>2
   acctgggaca actggaatcc 20
<210>3
   <211>22
   <212>DNA
   <213>Artificial Sequence
<220>
   <223>fibrillin-1 forward primer
<400>3
   gtgagatcaa catcaatgga gc 22
<210>4
   <211>23
   <212>DNA
   <213>Artificial Sequence
<220>
   <223>fibrillin-1 reverse primer
<400>4
   ttacacactc ctgggaacac ttc 23
<210>5
   <211>25
   <212>DNA
   <213>Artificial Sequence
<220>
   <223>fiblin-1 forward primer
<400>5
   gatgtcctcc tggaggcctg ctgtg 25
<210>6
   <211>26
   <212>DNA
   <213>Artificial Sequence
<220>
   <223>fiblin-1 reverse primer
<400>6
   ttgggtcggc agcggaagga tcccag 26
<210>7
   <211>20
   <212>DNA
   <213>Artificial Sequence
<220>
   <223>DANCE forward primer
<400>7
   cggcacatac ttctgctcct 20
<210>8
   <211>20
   <212>DNA
   <213>Artificial Sequence
<220>
   <223>DANCE reverse primer
<400>8
   tcagaatggg tactgcgaca 20
<210>9
   <211>19
   <212>DNA
   <213>Artificial Sequence
<220>
   <223>EMILIN forward primer
<400>9
   attatgacca gagacaggc 19
<210>10
   <211>20
   <212>DNA
   <213>Artificial Sequence
<220>
   <223>EMILIN reverse primer
<400>10
   ccgagtgcgc cagctgcccc 20
<210>11
   <211>21
   <212>DNA
   <213>Artificial Sequence
<220>
   <223>MFAP-2 forward primer
<400>11
   atgagagctg cctacctctt c 21
<210>12
   <211>21
   <212>DNA
   <213>Artificial Sequence
<220>
   <223>MFAP-2 reverse primer
<400>12
   ctagcagctc ccacagctcc t 21
<210>13
   <211>24
   <212>DNA
   <213>Artificial Sequence
<220>
   <223>GAPDH forward primer
<400>13
   tggtatcgtg gaaggactca tgac 24
<210>14
   <211>24
   <212>DNA
   <213>Artificial Sequence
<220>
   <223>GAPDH reverse primer
<400>14
   atgccagtga gcttcccgtt cagc 24